# EUROPÄISCHE PATENTSCHRIFT

(11) **EP 1 592 893 B1**
(45) Veröffentlichungstag und Bekanntmachung des Hinweises auf die Patenterteilung: **24.01.2007**
(21) Anmeldenummer: 03700801.8
(22) Anmeldetag: 12.02.2003
(51) Int. Cl.: F16B 31/02, A61B 17/86, A61B 17/80, A61B 17/88

(54) **SCHRAUBE MIT INTEGRIERTEM SCHRAUBENDREHER**
SCREW COMPRISING AN INTEGRATED SCREWDRIVER
VIS A TOURNEVIS INTEGRE

(43) Veröffentlichungstag der Anmeldung: 09.11.2005
(73) Patentinhaber: Synthes GmbH, 4436 Oberdorf (CH)
(72) Erfinder: MATTHYS-MARK, Romano, CH-7272 Davos Clavadel (CH)
(74) Vertreter: Lusuardi, Werther
(86) Internationale Anmeldenummer: PCT/CH2003/000101
(87) Internationale Veröffentlichungsnummer: WO 2004/072496

(56) Entgegenhaltungen:
- EP-A- 0 323 429
- FR-A- 2 721 819
- FR-A- 2 760 628
- US-A- 5 709 686

## Beschreibung

Die Erfindung bezieht sich auf eine Schraube mit integriertem Schraubendreher gemäss dem Oberbegriff des Patentanspruchs 1.

Insbesondere bei Schrauben mit kleinen Abmessungen, wie sie beispielsweise in der Osteosynthese Verwendung finden, stellt sich das Problem der sicheren Handhabung, des Haltens und des Führens. Bereits das Ergreifen kleinerer Schrauben, im besonderen bei anspruchsvollen Aufgaben, wie sie sich beispielsweise in der Knochenchirurgie stellen, kann sich äusserst schwierig gestalten.

Ein weiteres Problem besteht darin, dass mit gewöhnlichen Schraubendrehern keine Kontrolle des Drehmomentes möglich ist. Um ein im voraus bestimmtes Drehmoment anwenden zu können, sind spezielle Drehmomentschraubendreher notwendig.

Eine Schraube gemäss dem Oberbegriff des Anspruchs 1 ist beispielsweise aus dem Dokument FR 2 721 819 bekannt.

Hier will die Erfindung Abhilfe schaffen. Der Erfindung liegt die Aufgabe zugrunde, eine winkelstabil verankerbare Schraube mit einem über eine Sollbruchstelle integrierten Schraubendreher zu schaffen, so dass die Schraube mit einem vorbestimmten Drehmoment, einfach und sicher winkelstabil eingedreht werden kann.

Die Erfindung löst die gestellte Aufgabe mit einer Schraube, welche die Merkmale des Anspruchs 1 aufweist.

Durch das am Schraubenkopf angebrachte Aussengewinde, welches mit einem entsprechenden (kreiszylindrischen oder konischen) Innengewinde in der Plattenbohrung korrespondiert wird sichergestellt, dass die Platte nicht an den Knochen herangepresst wird und die Schraube sicher und winkelstabil in der Platte verankert ist und auch bleibt. Dies steht im Gegensatz zu einer glatten konischen Ausführung des Schraubenkopfes, bei welcher der Kopf nur auf Grund der Reibkraft festsitzt und somit keinen Formschluss aufweist. Der nur auf der Reibkraft basierende Reibschluss entsteht durch Gegenhalt vom Knochen beim Festziehen der Schraube und hat den Nachteil, dass er sich durch Belastung der Schraube wiederum lösen kann.

Beim Festziehen der Schraube wird der integrierte Schraubendreher an der Sollbruchstelle abgeschert, wobei das Abschermoment durch die gewählte Dimensionierung der Sollbruchstelle definiert ist.

Die erfindungsgemässe Schraube mit integriertem Schraubendreher gestattet auch sehr klein dimensionierte Schrauben einfach zu halten und gut geführt in eine Bohrung, z.B. einer Knochenplatte einzudrehen. Durch die koaxiale Anordnung des integrierten Schraubendrehers und seine feste Verbindung mit dem Schraubenkopf, kann die Schraube in jeder beliebigen Position um die Längsachse aufgenommen werden und es muss nicht auf die Position des Schraubendrehers Rücksicht genommen werden.

Weitere vorteilhafte Ausgestaltungen der Erfindung sind in den abhängigen Ansprüchen gekennzeichnet.

Bei einer besonderen Ausführungsform erweitert sich der Schraubenkopf in Richtung des Schraubendrehers konisch, was eine winkelstabile Fixierung des Schraubenkopfs in einer Knochenplatte mit entsprechender konischer Plattenbohrung erlaubt.

Vorteilhafterweise ist das Gewinde des Schraubenschaftes selbstschneidend ausgebildet, indem an seinem freien Ende, das heisst an der Schraubenspitze, eine entsprechende Nut angebracht ist, welche im wesentlichen parallel zur Längsachse der Schraube verläuft.
Die erfindungsgemässe Vorrichtung eignet sich besonders für klein dimensionierte Schrauben, typischerweise solchen mit einen Durchmesser kleiner als 2,0 mm, vorzugsweise kleiner als 1,0 mm.

Die Sollbruchstelle sollte vorteilhafterweise einen Durchmesser aufweisen, der kleiner ist als der Durchmesser des zwischen dem Schraubenschaft und dem Schraubenkopf angeordneten Gewindefreistichs.

Bei einer besonderen Ausführungsform weist die Sollbruchstelle einen Durchmesser von 60 bis 82 %, vorzugsweise von 66 bis 75 % des Aussendurchmessers des Gewindes auf. In absoluten Zahlen kann die Sollbruchstelle einen Durchmesser von 0,25 bis 0,5 mm, vorzugsweise von 0,32 bis 0,38 mm aufweisen

Die Schraube mit integriertem Schraubendreher kann über eine Kupplung mit einem mechanischen Antrieb verbunden werden. Dieser Antrieb kann entweder ein manuell betätigbarer Handgriff oder eine motorische betätigbare Maschine sein.

Die Erfindung und Weiterbildungen der Erfindung werden im folgenden anhand einer teilweise schematischen Darstellungen eines Ausführungsbeispiels noch näher erläutert.

Es zeigt:
Fig. 1 eine perspektivische Ansicht der Schraube mit integriertem Schraubendreher;
Fig. 2 einen Längsschnitt durch die Schraube mit integriertem Schraubendreher; und
Fig. 3 eine perspektivische Ansicht einer Knochenplatte zur Aufnahme einer Schraube nach Fig. 1.

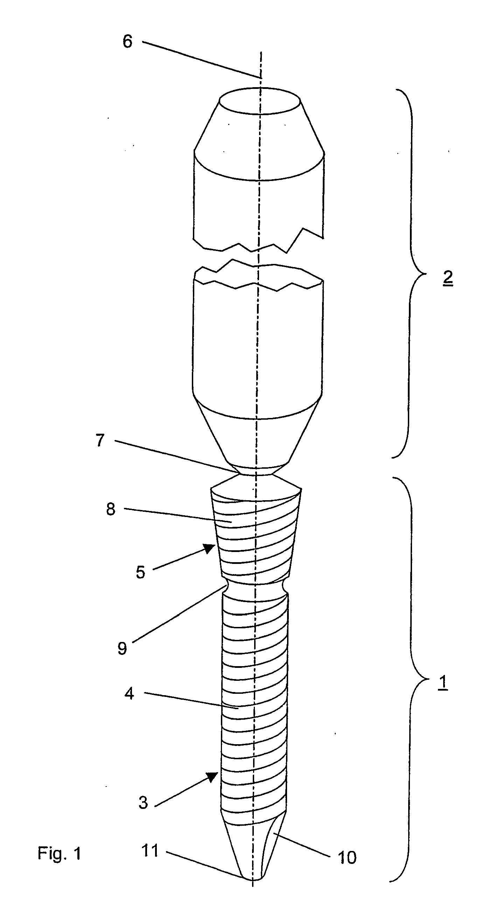

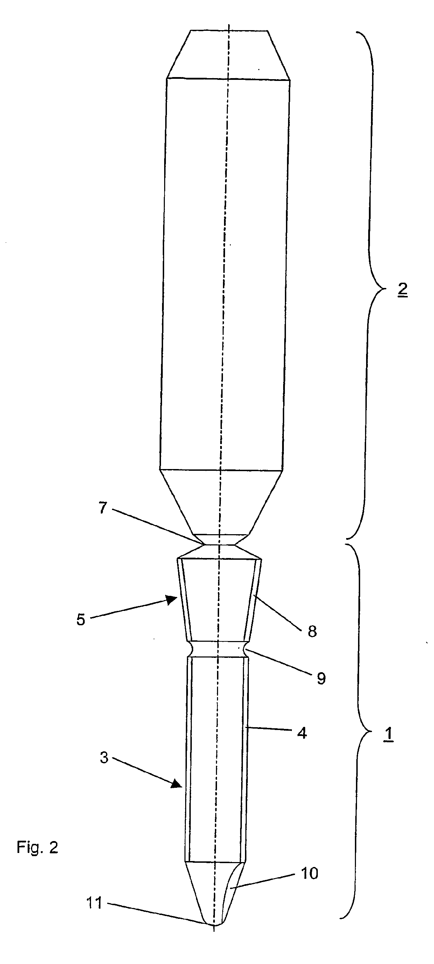

Die in den Fig. 1 und 2 dargestellte Schraube 1 und der integrierte Schraubendreher 2 weisen eine gemeinsame Längsachse 6 auf. Die als Knochenschraube ausgebildete Schraube 1 weist eine Länge von 2,6 mm auf und besitzt einen Schraubenschaft 3 der Länge 1,3 mm mit einem Gewinde 4 und einen konischen Schraubenkopf 5 der Länge 1,1 mm mit Aussengewinde 8. Zwischen Schraubenschaft 3 und Schraubenkopf 5 ist ein Gewindefreistich 9 angeordnet. Das Gewinde 4 des Schraubenschaftes 3 ist mittels einer Nut 10 selbstschneidend ausgebildet. Der Schraubendreher 2 ist 4,9 mm lang und über eine Sollbruchstelle 7 koaxial mit dem Schraubenkopf 5 verbunden. Die Sollbruchstelle weist einen Durchmesser von 0,35 mm auf.

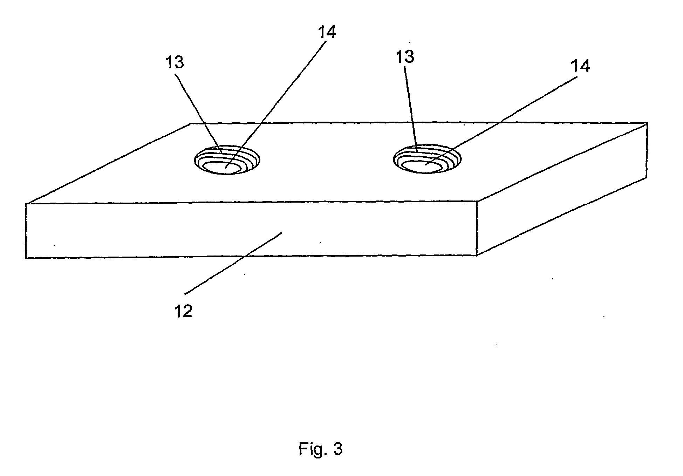

Die Schraube 1 kann in die Plattenbohrung 13 der in Fig. 3 dargestellten Knochenplatte 12 eingeführt werden. Das zum Aussengewinde 8 der Schraube 1 korrespondierende Innengewinde 14 der Plattenbohrung 13 bewirkt eine winkelstabile Verankerung der Schraube 1 bezüglich der Knochenplatte 12. Das Innengewinde 14 und das dazu korrespondierende Aussengewinde 8 können auch mehrgängig, vorzugsweise zweigängig, ausgebildet sein.

## Patentansprüche

1. Schraube (1) mit integriertem Schraubendreher (2), wobei
A) die Schraube (1) einen Schraubenschaft (3) mit Gewinde (4), einen Schraubenkopf (5) und eine Längsachse (6) aufweist;
B) der Schraubendreher (2) koaxial zur Längsachse (6) über eine Sollbruchstelle (7) mit dem Schraubenkopf (5) verbunden ist; und
C) der Schraubenkopf (5) sich in Richtung des Schraubendrehers (2) konisch erweitert;
**dadurch gekennzeichnet, dass**
D) der Schraubenkopf (5) ein Aussengewinde (8) trägt; und dass
E) zwischen dem Schraubenschaft (3) und dem Schraubenkopf (5) ein Gewindefreistich (9) angeordnet ist.

2. Schraube (1) mit integriertem Schraubendreher (2) nach Anspruch 1, **dadurch gekennzeichnet, dass** das Gewinde (4) des Schraubenschaftes (3) selbstschneidend ausgebildet ist.

3. Schraube (1) mit integriertem Schraubendreher (2) nach Anspruch 1 oder 2, **dadurch gekennzeichnet, dass** an ihrem freien Ende (11) eine Nut (10) angebracht ist.

4. Schraube (1) mit integriertem Schraubendreher (2) nach einem der Ansprüche 1 bis 3, **dadurch gekennzeichnet, dass** die Schraube (1) eine Knochenschraube ist.

5. Schraube (1) mit integriertem Schraubendreher (2) nach einem der Ansprüche 1 bis 4, **dadurch gekennzeichnet, dass** die Schraube (1) einen Durchmesser kleiner als 2,0 mm aufweist.

6. Schraube (1) mit integriertem Schraubendreher (2) nach Anspruch 5, **dadurch gekennzeichnet, dass** die Schraube (1) einen Durchmesser kleiner als 1,0 mm aufweist.

7. Schraube (1) mit integriertem Schraubendreher (2) nach einem der Ansprüche 1 bis 6, **dadurch gekennzeichnet, dass** die Sollbruchstelle (7) einen Durchmesser aufweist, der kleiner ist als der Durchmesser des Gewindefreistichs.

8. Schraube (1) mit integriertem Schraubendreher (2) nach einem der Ansprüche 1 bis 7, **dadurch gekennzeichnet, dass** die Sollbruchstelle (7) einen Durchmesser von 60 bis 82 %, vorzugsweise von 66 bis 75 % des Aussendurchmessers des Gewindes (4) des Schraubenschaftes (3) aufweist.

9. Schraube (1) mit integriertem Schraubendreher (2) nach einem der Ansprüche 1 bis 8, **dadurch gekennzeichnet, dass** die Sollbruchstelle (7) einen Durchmesser von 0,25 bis 0,5 mm, vorzugsweise von 0,32 bis 0,38 mm aufweist.

10. Schraube (1) mit integriertem Schraubendreher (2) nach einem der Ansprüche 1 bis 9, **dadurch gekennzeichnet, dass** das Aussengewinde (8) des Schraubenschaftes (5) mehrgängig, vorzugsweise zweigängig ausgebildet ist,

11. Knochenplatte (12) mit mindestens einer Schraube (1) nach einem der Ansprüche 1 bis 10,
**dadurch gekennzeichnet, dass**
die Knochenplatte mindestens eine Plattenbohrung (13) aufweist, welche mit einem zum Gewinde (4) des Schraubenschaftes (3) korrespondierenden Innengewinde (14) versehen ist.

## Claims

1. Screw (1) with an integrated screw driver (2), whereby
A) the screw (1) is provided with a threaded shaft (3) with thread (4), a screw head (5) and a longitudinal axis (6);
B) the screw driver (2) is connected to the screw head (5) coaxially to the longitudinal axis (5) via a predetermined breaking point; and
C) the screw head (5) conically enlarges towards the screw driver (2),
**characterized in that**
D) the screw head (5) is provided with an external thread (8); and that
E) an undercut (9) is disposed between the threaded shaft (3) and the screw head (5).

2. Screw (1) with an integrated screw driver (2) of claim 1, wherein the thread (4) of the threaded shaft (3) is self-cutting.

3. Screw (1) with an integrated screw driver (2) of claim 1 or 2, wherein at its free end (11) a groove (10) is provided.

4. Screw (1) with an integrated screw driver (2) of one of claims 1 to 3, wherein the screw (1) is a bone screw.

5. Screw (1) with an integrated screw driver (2) of one of claims 1 to 4, wherein the screw (1) has a diameter of less than 2,0 mm.

6. Screw (1) with an integrated screw driver (2) of claim 5, wherein the screw (1) has a diameter of less than 1,0 mm.

7. Screw (1) with an integrated screw driver (2) of one of claims 1 to 6, wherein the predetermined breaking point (7) has a diameter which is smaller than the diameter of the undercut.

8. Screw (1) with an integrated screw driver (2) of one of claims 1 to 7, wherein the predetermined breaking point (7) has a diameter from 60 to 82%, preferably 66 to 75% of the external diameter of the thread (4) of the threaded shaft (3).

9. Screw (1) with an integrated screw driver (2) of one of claims 1 to 8, wherein the predetermined breaking point (7) has a diameter from 0,25 to 0,5 mm, preferably from 0,32 to 0,38 mm.

10. Screw (1) with an integrated screw driver (2) of one of claims 1 to 9, wherein the external thread (8) of the threaded shaft (3) is a multiple thread, preferably a double thread.

11. Bone plate (12) with at least one screw (1) according to one of the claims 1 to 10,
**characterized in that**
the bone plate is provided with at least one plate borehole (13) which is provided with an internal thread (14) corresponding to the external thread (4) of the threaded shaft (3).

## Revendications

1. Vis (1) avec tournevis intégré (2), dans laquelle
A) la vis (1) présente une tige de vis (3) avec un filetage (4), une tête de vis (5) et un axe longitudinal (6) ;
B) le tournevis (2) est relié à la tête de vis (5) coaxialement à l'axe longitudinal (6) par l'intermédiaire d'un point de rupture (7) ; et
C) la tête de vis (5) s'élargit de manière conique en direction du tournevis (2) ;
**caractérisée en ce que**
D) la tête de vis (5) porte un filetage extérieur (8) ; et **en ce que**
E) une gorge de dégagement (9) est disposée entre la tige de vis (3) et la tête de vis (5).

2. Vis (1) avec tournevis intégré (2) selon la revendication 1, **caractérisée en ce que** le filetage (4) de la tige de vis (3) est réalisé sous une forme autotaraudeuse.

3. Vis (1) avec tournevis intégré (2) selon la revendication 1 ou 2, **caractérisée en ce qu'**une encoche (10) est prévue sur son extrémité libre (11).

4. Vis (1) avec tournevis intégré (2) selon l'une quelconque des revendications 1 à 3, **caractérisée en ce que** la vis (1) est une vis à os.

5. Vis (1) avec tournevis intégré (2) selon l'une quelconque des revendications 1 à 4, **caractérisée en ce que** la vis (1) présente un diamètre inférieur à 2,0 mm.

6. Vis (1) avec tournevis intégré (2) selon la revendication 5, **caractérisée en ce que** la vis (1) présente un diamètre inférieur à 1,0 mm.

7. Vis (1) avec tournevis intégré (2) selon l'une quelconque des revendications 1 à 6, **caractérisée en ce que** le point de rupture (7) présente un diamètre qui est inférieur au diamètre de la gorge de dégagement.

8. Vis (1) avec tournevis intégré (2) selon l'une quelconque des revendications 1 à 7, **caractérisée en ce que** le point de rupture (7) présente un diamètre représentant 60% à 82%, de préférence 66% à 75% du diamètre extérieur du filetage (4) de la tige de vis (3).

9. Vis (1) avec tournevis intégré (2) selon l'une quelconque des revendications 1 à 8, **caractérisée en ce que** le point de rupture (7) présente un diamètre de 0,25 à 0,5 mm, de préférence de 0,32 à 0,38 mm.

10. Vis (1) avec tournevis intégré (2) selon l'une quelconque des revendications 1 à 9, **caractérisée en ce que** le filetage extérieur (8) de la tige de vis (3) est réalisé sous forme multiple, de préférence sous forme double.

11. Plaque d'ostéosynthèse (12) avec au moins une vis (1) selon l'une quelconque des revendications 1 à 10, **caractérisée en ce que** la plaque d'ostéosynthèse présente au moins un alésage de plaque (13), lequel est pourvu d'un taraudage (14) correspondant au filetage (4) de la tige de vis (3).
